# EUROPEAN PATENT APPLICATION

(11) **EP 2 546 333 A2**
(43) Date of publication of application: **16.01.2013**
(21) Application number: 12177594.4
(22) Date of filing: 08.11.2005
(51) Int. Cl.: C12N 5/00

(54) **Cardiac stem cells**

(30) Priority: 08.11.2004 US 625695 P
(62) Divisional of application: 05817349.3
(71) Applicant: Johns Hopkins University, Baltimore, MD 21201 (US)
(72) Inventor: Marban, Eduardo, Beverly Hills, CA 90210 (US); Abraham, Maria, Baltimore, MD Maryland 21210 (US); Smith, Rachel, Baltimore, Maryland 21224 (US)
(74) Representative: Tombling, Adrian George

(57) **Abstract**

Human cardiac stem cells can be isolated from endomyocardial biopsies. Such cells mediate cardiac regeneration and improve heart function in a mouse infarct model. The cells can be used for autologous, allogeneic, syngeneic, or xenogeneic therapeutic applications in patients. The stem cells can be genetically modified to enhance their therapeutic activity.

## Description

This application claims the benefit of provisional applications serial numbers 60/625,695 filed November 08, 2004, the disclosure of which is expressly incorporated herein.

### TECHNICAL FIELD OF THE INVENTION

This invention is related to the area of adult stem cells. In particular, it relates to harvesting, expansion, and reintroduction of stem cells.

### BACKGROUND OF THE INVENTION

Until recently, prevailing dogma posited that the heart is a terminally-differentiated organ with no regenerative potential. That view was undermined by the demonstration that the adult heart contains a small population of endogenous committed cardiac stem cells (CSCs), also known as cardiac progenitor cells, identifiable by their surface expression of c-Kit, MDR1, or Sca-1 (1-5). CSCs represent a logical cell source to exploit for cardiac regenerative therapy. Their expression of early cardiac transcription factors, and capability for ex vivo and in vivo differentiation toward the cardiac lineages, offer the prospect of enhanced cardiogenicity compared to other cell sources. CSCs can be isolated from human surgical samples without selective pressure and expanded in primary culture (6).

Cardiac surgical biopsies in culture yield spherical, multi-cellular clusters dubbed "cardiospheres" (6). Such cardiospheres are intriguingly cardiac-like in their expression of key myocardial structural proteins; when injected into the hearts of mice, human cardiospheres regenerated myocardium and vasculature in vivo.

There is a continuing need in the art for a simple, non-surgical method for harvesting and expansion of human CSCs for subsequent autologous, allogeneic, syngeneic, or xenogeneic transplantation.

### SUMMARY OF THE INVENTION

According to one embodiment of the invention a method is provided for increasing function of a damaged or diseased heart of a mammal. A population of cells is administered to the mammal. The population of cells thereby increases cardiac function in the mammal. The population of cells is obtained by the process of culturing cells obtained from cardiospheres on a surface as a monolayer.

Another embodiment of the invention provides a method for increasing function of a damaged or diseased heart of a mammal. A population of in vitro-expanded cells is administered to the mammal. The cells have the capacity to form cardiospheres in suspension culture. The cells are not, however, in the form of cardiospheres when administered.

Yet another embodiment of the invention provides a method of treating a mammal with a damaged or diseased heart. Heart tissue is obtained from the damaged or diseased heart of the mammal or from a healthy heart of a donor via a percutaneous endomyocardial biopsy. The heart tissue is treated to obtain and expand a population of cardiac stem cells. The cardiac stem cells and/or their progeny are introduced into the damaged or diseased heart of the mammal.

According to another embodiment of the invention a method of treating a cardiac biopsy specimen is provided. The cardiac biopsy specimen is incubated in the presence of a protease. The cells liberated from the biopsy specimen by the protease incubation are collected. The collected cells are cultured on a surface as a monolayer to expand number of cells.

Another aspect of the invention is a method of treating a mammal with a damaged or diseased organ. Tissue is obtained from the damaged or diseased organ of the mammal or from a healthy organ of a donor via a percutaneous biopsy. The tissue is treated to obtain and expand a population of stem cells. The stem cells and/or their progeny are introduced into the damaged or diseased organ of the mammal.

A further aspect of the invention is a method for expanding a population of cardiac stem cells. One or more cardiospheres are disaggregated to individual cells or smaller aggregates of cells. The individual cells or smaller aggregates of cells are cultured on a surface as a monolayer.

The invention also provides a population of in vitro-expanded cells in a monolayer. The cells have the capacity to form cardiospheres in suspension culture. The cells are not, however, in the form of cardiospheres.

Still another aspect of the invention is a population of cells made by the process of culturing cells on a surface as a monolayer. The cells are obtained from disaggregated cardiospheres.

A further ramification of the invention is a method of treating a kidney biopsy specimen. The kidney biopsy specimen is incubated in the presence of a protease. The cells liberated from the biopsy specimen by the protease incubation are collected. The collected cells are cultured on a surface as a monolayer to expand number of cells.

These and other embodiments which will be apparent to those of skill in the art upon reading the specification provide the art with methods and populations for therapy of diseased and damaged organs.

### BRIEF DESCRIPTION OF THE DRAWINGS

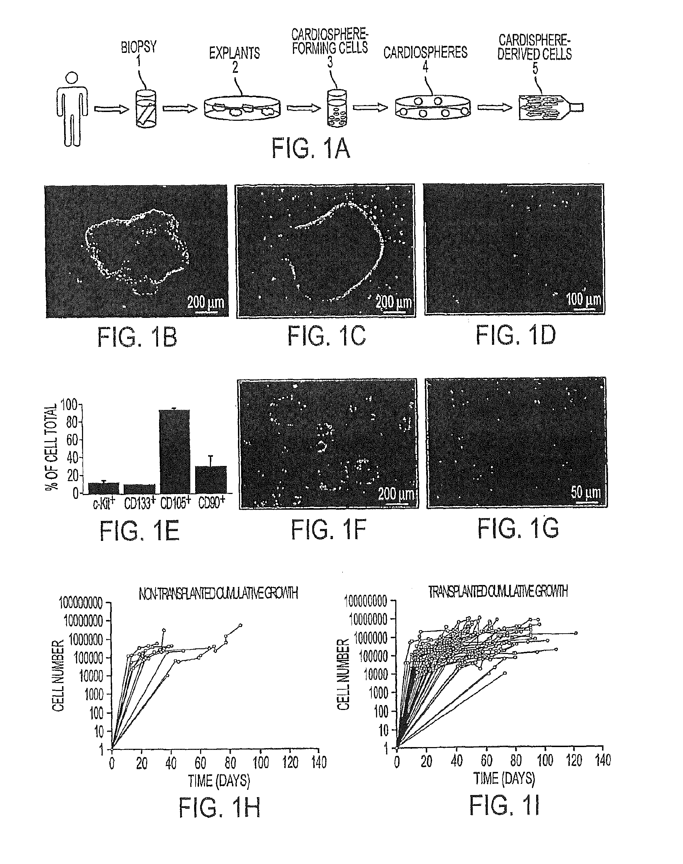

*Figures 1(A)-1(I)**. Specimen Processing for Cardiosphere Growth and Cardiosphere-Derived Cell (CDC) Expansion.* Fig. 1A schematic depicts the steps involved in specimen processing. Fig. 1B) Human endomyocardial biopsy fragment on day 1. Fig. 1C) Human explant 3 days after plating. Fig. 1D) Edge of human explant on 13 days after plating showing stromal-like and phase-bright cells. Fig. 1E) Results of sub-population selection performed using cardiosphere-forming cells. c-Kit⁺ cells were 90.0±4.7% CD105⁺, and c-Kit⁻ cells were 94.0±0.8% CD105⁺ (n=3). Fig. 1F) Human cardiospheres on day 25, 12 days after collection of cardiosphere-forming cells. Fig. 1G) Human CDCs during passage 2, plated on fibronectin for expansion. Fig. 1H) Cumulative growth for 11 specimens from untransplanted patients is depicted over the course of 4 months. Fig. 1 I) Growth for 59 specimens from transplanted patients is shown. Day 0 corresponds to the date the specimen was collected and cell number on that day is plotted as 1 on the log scale, since no cardiosphere-forming cells had yet been harvested from the specimen.

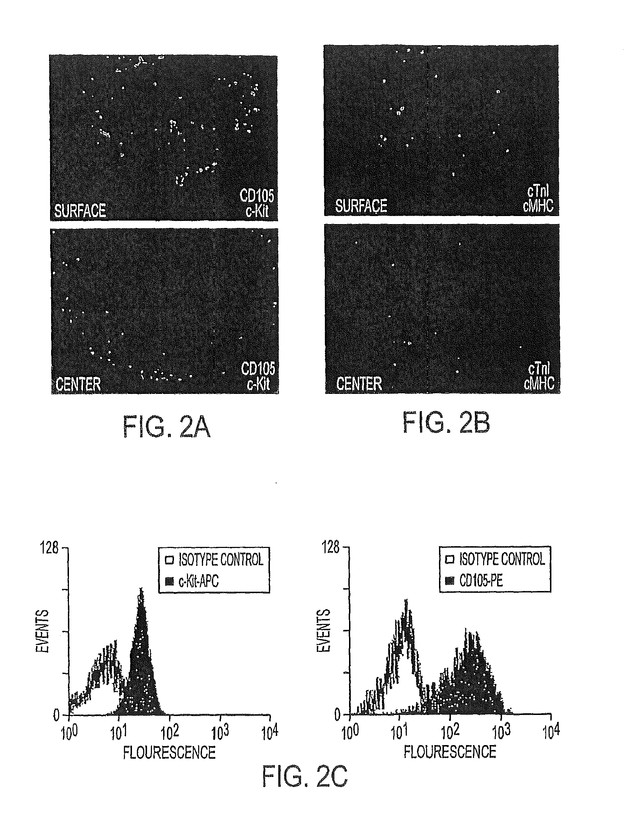

*Figures 2A-2C**. Cardiosphere and CDC Phenotypes.* Fig. 2A) Cardiosphere expressing c-Kit throughout its core and CD105 on its periphery. Fig. 2B) Cardiosphere expressing cardiac MHC and TnI primarily on its periphery. Fig. 2C) c-Kit and CD105 expression levels in CDCs at passage 2 shown for one representative specimen (n=3 and n=2).

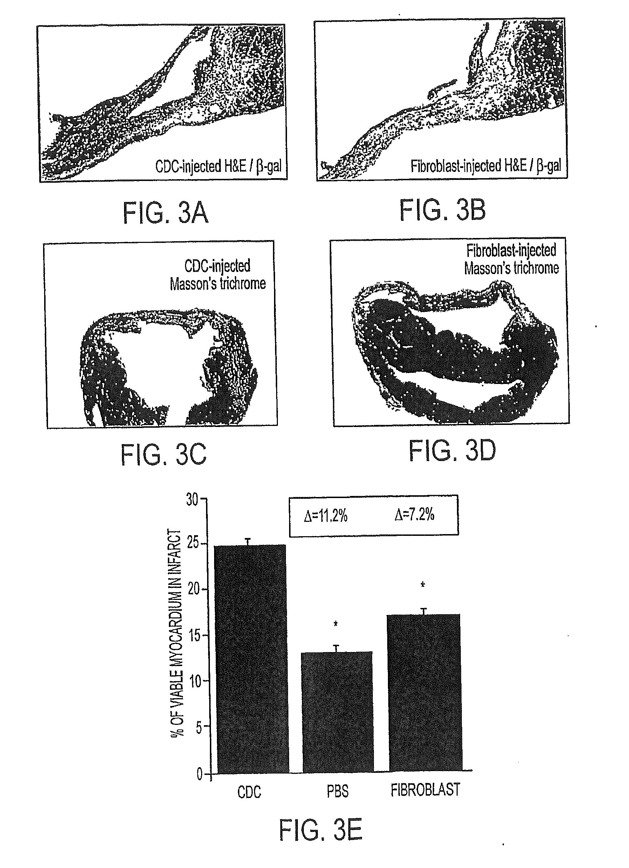

*Figures 3A-3E**. Engraftment and Regeneration.* Fig. 3A and Fig. 3B) Engraftment of CDCs (Fig. 3A) or fibroblasts (Fig. 3 B) is depicted 20 days after injection in heart sections double stained for H&E and β-galactosidase. Infiltration of CDCs is seen as a distinct band, while a rare group of a few fibroblasts can be detected in some sections. Fig. 3C and Fig. 3D) Masson's trichrome staining as used to calculate myocardial regeneration is shown for a representative CDC-injected mouse (Fig. 3C) and fibroblast-injected mouse (Fig. 3D). Fig. 3 E) The percent of viable myocardium found within the infarcted area in CDC (n=8), PBS (n=4), and fibroblast-injected (n=4) groups is shown. * p<0.01.

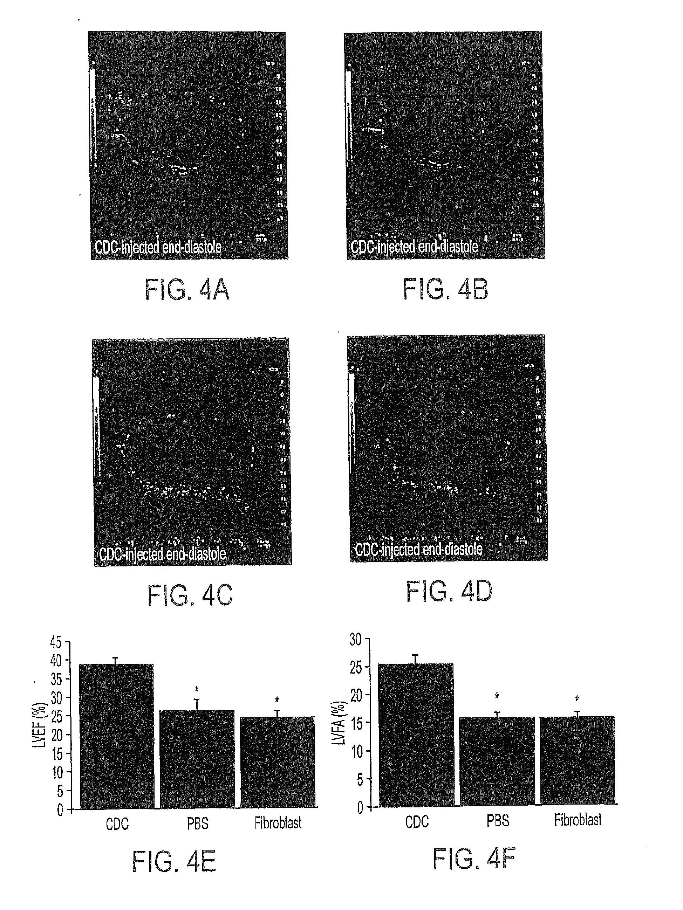

*Figures 4A-4F**. Functional Improvement.* Fig. 4A and Fig. 4B) Long-axis views from an echocardiogram performed after 20 days in a CDC-injected mouse. Fig. 4A shows end-diastole. Fig. 4B shows end-systole. Yellow lines trace around the left ventricular area used for the calculation of LVEF and LVFA. Fig. 4C and Fig. 4D) Comparable views in a fibroblast-injected mouse. Fig. 4E) Left ventricular ejection fractions for the three experimental groups after 20 days (CDC n=8, PBS n=7, Fibroblast n=4; * p<0.01). LVEF=100x(LVVolume_{diastole}-LVVolume_{systole})/LVVolume_{diastole}, where LVVolume was calculated from long-axis views assuming a prolate ellipsoid. Fig. 4F) Left ventricular percent fractional area for the three experimental groups after 20 days. * p<0.01. LVFA=100x(LVArea_{diastole}-LVArea_{systole})/LVArea_{diastole}.

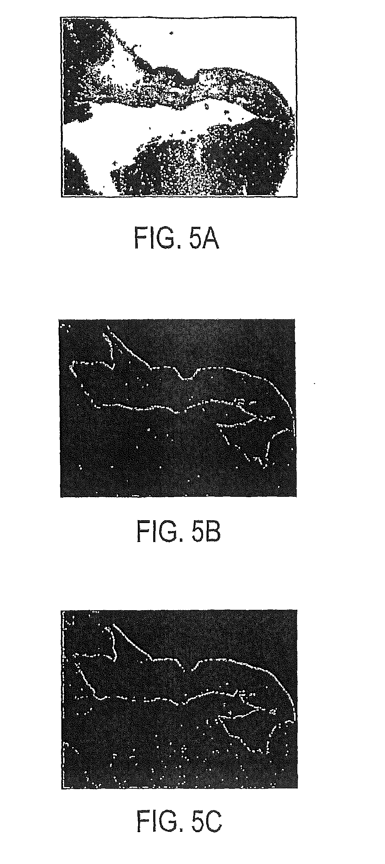

*Figure 5A-5C**. Quantifying Regeneration.* Fig. 5A) Masson's trichrome staining is shown for a representative CDC-injected mouse. The total infarct zone is outlined in yellow in Fig. 5B and Fig. 5C. Fig. 5B) Areas of fibrosis are shown in red after image processing. Fig. 5C) Areas of viable myocardium are shown in red after image processing. Six sections were analyzed per animal and an average taken.

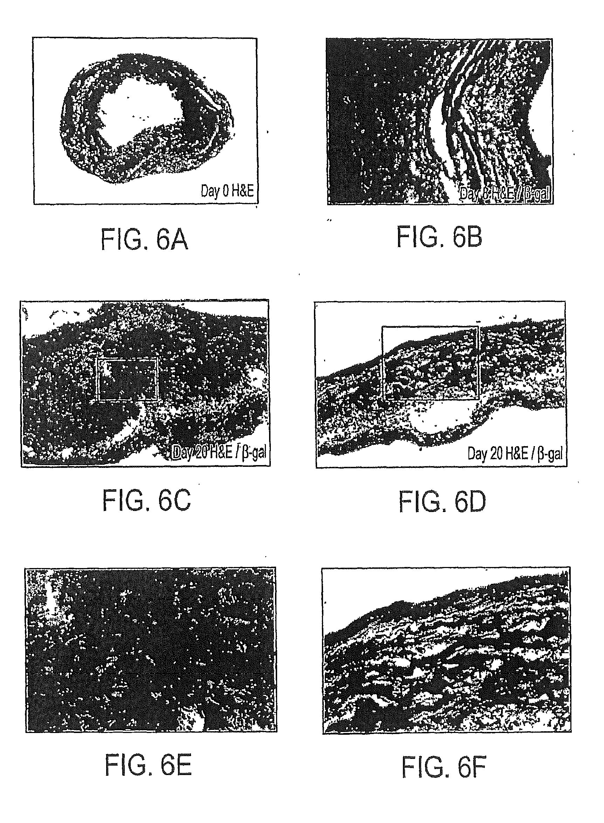

*Figure 6A-6F**. Engraftment Timecourse.* Fig. 6A) The bolus of injected cells is shown on day 0 in an H&E stained section. Fig. 6B) Engraftment of CDCs is depicted 8 days after injection. Fig. 6C and Fig. 6D) Engraftment of CDCs 20 days after injection. Fig. 6E and Fig. 6F) Corresponding higher magnification views of Fig. 6C and Fig. 6D demonstrating colocalization of lac-Z-positive CDCs and viable myocardium.

### DETAILED DESCRIPTION OF THE INVENTION

The inventors have developed methods for expanding populations of resident stem cells from organs, such that only small initial samples are required. Such small initial samples can be obtained relatively non-invasively, by a simple percutaneous entry. Such procedures are so simple that that they can be done on an out-patient basis without major surgery or general anesthesia.

Resident stem cells are those which are found in a particular organ. Although applicants do not wish to be bound by any particular theory, it is believed that the stem cells found in a particular organ are not pluripotent, but rather, are committed to a particular branch of differentiation. Thus in the heart, one expects to find cardiac stem cells, and in the kidney one expects to find kidney stem cells. Nonetheless, it is possible that some of the stem cells expanded and isolated by the present invention are able to develop into cells of an organ other than the one from which they were obtained.

Cardiospheres are self-associating aggregates of cells which have been shown to display certain properties of cardiomyocytes. Thus cardiospheres have been shown to "beat" in vitro. They are excitable and contract in synchrony. The cells which form the cardiospheres have been obtained from heart biopsies. The cardiospheres can be disaggregated using standard means known in the art for separating cell clumps or aggregates, including, but not limited to trituration, agitation, shaking, blending. Preferably the cardiospheres are disaggregated to single cells, but at least they are disaggregated to smaller aggregates of cells. After disaggregation, the cells can be grown on a solid surface, such as a culture dish, a vessel wall or bottom, a microtiter dish, a bead, flask, roller bottle, etc. The surface can be glass or plastic, for example. The cells can adhere to the material of the solid surface or the solid surface can be coated with a substance which encourages adherence. Such substances are well known in the art and include, without limitation, fibronectin. hydrogels, polymers, laminin, serum, collagen, gelatin, and poly-L-lysine. Growth on the surface will preferably be monolayer growth.

After growth of disaggregated cells, they can be directly administered to a mammal in need thereof, or they can be grown under conditions which favor formation of cardiospheres. Repeated cycling between surface growth and suspension growth (cardiospheres) leads to a rapid and exponential expansion of desired cells. One can also eliminate the cardiosphere phase and repeatedly expand cells which are grown on a surface without forming cardiospheres at each passage.

The cell culturing of the present invention, whether on cell surfaces or in cardiospheres can be performed in the absence of exogenous growth factors. While fetal bovine serum can be used, other factors have been found to be expendable. For example the cells of the present invention are readily cultured in the absence of added EGF, bFGF, cardiotrophin-1, and thrombin.

Mammals which can be the donors and recipients of cells are not limited. While humans can provide both the cells and be the recipients, often other mammals will be useful. Pig cells can be transplanted into humans, for example. Such cross-species transplantation is known as xenogeneic transplantation. The transplantation can also be allogeneic, syngeneic, or autologous, all within a single species. Suitable mammals for use in the present invention include pets, such as dogs, cats, rabbits; agricultural animals, such as horses, cows, sheep, goats, pigs; as well as humans.

Administration of cells to a mammal can be by any means known in the art. Cardiac cells can be delivered systemically or locally to the heart. The cells are typically not in the form of cardiospheres. Typically they have the capacity to form cardiospheres, however, under suitable conditions. Local administration can be by catheter or during surgery. Systemic administration can be by intravenous or intraarterial injections, perfusion, or infusion. When the populations of cells of the invention are administered systemically, they migrate to the appropriate organ, e.g., the heart, if the cells are derived from resident heart stem cells. The beneficial effects which are observed upon administration of the cells to a mammal may be due to the cells *per se,* or due to products which are expressed by the cells. For example, it is possible that the engraftment of cells produces a favorable outcome. It is also possible that cytokines or chemokines or other diffusible factors stimulate resident cells to grow, reproduce, or perform better.

An effective dose of cardiac stem cells will typically be between 1 x 10⁶ and 100 x 10⁶, preferably between 10 x 10⁶ and 50 x 10⁶. Depending on the size of the damaged region of the heart, more or less cells can be used. A larger region of damage may require a larger dose of cells, and a small region of damage may require a smaller does of cells. On the basis of body weight of the recipient, an effective dose may be between 1 and 10 x 10⁶ per kg of body weight, preferably between 1 x 10⁶ and 5 x 10⁶ cells per kg of body weight.. Patient age, general condition, and immunological status maybe used as factors in determining the dose administered.

Diseases which can be treated according to the present invention include acute and chronic heart disease. For example, the heart may have been subjected to an ischemic incident, or may be the subject of chronic ischemia or congestive heart disease. Patients may be candidates for heart transplants or recipients of heart transplants. In addition, hearts which are damaged due to trauma, such as damage induced during surgery or other accidental damage, can be treated with cells according to the invention.

Because of the excellent expansion of cell populations achieved, the initial cell samples need not be large. Thus, rather than starting with a conventional biopsy sample, obtained during surgery, a smaller sample can be used which eliminates the need for invasive surgery. Such samples can be obtained using a percutaneous bioptome. The bioptome can be used to access a tissue sample from any organ source, including heart, kidney, liver, spleen, and pancreas. Particularly suitable locations within the heart which can be accessed using a bioptome include the crista terminalis, the right ventricular endocardium, the septal or ventricle wall, and the atrial appendages. These locations have been found to provide abundant stem or progenitor cells. Accessing such locations is facilitated by use of a bioptome which is more flexible than the standard bioptome used for accessing the right ventricular endocardium for diagnostic purposes. Preferably the bioptome is also steerable by an external controller.

One of the enhancements that has led to the ability to use small biopsy samples as a starting material is the collection of a cell population which has previously been ignored or discarded. This cell population is formed by treating the biopsy sample with a protease and harvesting or collecting the cells that are liberated from the biopsy sample. The use of these liberated cells enhances the rate of cell population expansion. Examples of proteases which can be employed include collagenase, matrix metalloproteases, trypsin, and chymotrypsin. This technique can be applied to any organ from which resident stem cells are desired, including, for example, heart, kidney, lung, spleen, pancreas, and liver.

The cell populations which are collected, expanded, and/or administered according to the present invention can be genetically modified. They can be transfected with a coding sequence for a protein, for example. The protein can be beneficial for diseased organs, such as hearts. Examples of coding sequences which can be used include without limitation akt, connexin 43, other connexins, HIF1α, VEGF, FGF, PDGF, IGF, SCF, myocardin, cardiotrophin, L-type calcium channel α subunit, L-type calcium channel β subunit, and Nkx2.5. The cells may be conveniently genetically modified before the cells are administered to a mammal. Techniques for genetically modifying cells to express known proteins are well known in the art.

Cardiosphere-Derived Cells (CDCs) were easily harvested and readily expanded from biopsy specimens, and we have shown them to regenerate myocardium and improve function in an acute MI model. Remarkably, 69 of 70 patients had specimens that yielded cells by our method, making the goal of autologous cellular cardiomyoplasty attainable. Early clinical studies would logically focus on autologous cells, which are a perfect genetic match and thus present fewer safety concerns than allogeneic cells. A practical limitation with the use of autologous cells arises from the delay from tissue harvesting to cell transplantation. To avoid the delay, cell banks can be created of cardiac stem cells (CSCs) from patients with defined immunological features. These should permit matching of immunological antigens of donor cells and recipients for use in allogeneic transplantation. Antigens for matching are known in the art of transplantation.

Previous clinical studies in which bone marrow-derived stem cells were injected into patients within 2 weeks following acute MI, resulted in significantly improved LVEF with intracoronary infusion of 5-80x10⁶ cells (15-17), leaving us to postulate that several million CDCs may constitute an effective therapeutic dose. From single bioptome specimens, millions of CDCs can be derived after just two passages; if biopsies were performed specifically for therapeutic purposes, the amount of starting material could easily be scaled upwards by ten-fold or more, further improving the overall cell yield. Patients with chronic heart failure are also good candidates for CDC therapy.

Minimizing the number of passages for expansion will minimize the risk of cancerous transformation of CDCs, a problem which has been observed in mesenchymal stem cells, but only after >6 passages (18). Another prominent risk of cell transplantation lies in the potential for arrhythmogenicity (19-21). Arrhythmias have not been documented with cardiac stem cells.

We have used CDCs derived from human biopsies without antigenic selection. We have purposely included all cells that are shed from the initial heart specimen and which go on to contribute to the formation of cardiospheres. Thus, our cells differ fundamentally from cardiac "stem cells" which have been isolated by antigenic panning for one or another putative stem cell marker (2, 3). Nevertheless, CDCs include a sizable population of cells that exhibit stem cell markers, and the observed regenerative ability in vivo further supports the notion that CDCs include a number of resident stem cells. We do not yet know whether a subfraction of CDCs suffices to produce the beneficial effects; indeed, we have avoided subfractionation since it would likely delay transplantation and raise regulatory concerns by introducing an artificial selection step.

Adult human cardiac stem cells have been shown to respond to a limited degree to a state of cardiac hypertrophy by proliferation and myocardial regeneration (4) and to acute ischemia by mobilization to the injury border zone and subsequent regeneration, but often ultimately succumb to apoptosis in a chronic ischemic setting (5). Significant progress is currently being made identifying means of enhancing in vivo survival, mobilization, proliferation, and subsequent differentiation of CSCs using animal models (22, 23). Our method for ex vivo expansion of resident stem cells for subsequent autologous transplantation may give these cell populations, the resident and the expanded, the combined ability to mediate myocardial regeneration to an appreciable degree. If so, cardiac stem cell therapy may well change our fundamental approach to the treatment of disorders of cardiac dysfunction.

The above disclosure generally describes the present invention. All references disclosed herein are expressly incorporated by reference. A more complete understanding can be obtained by reference to the following specific examples which are provided herein for purposes of illustration only, and are not intended to limit the scope of the invention.

### EXAMPLE 1

### Materials and Methods:

### Specimen Processing and Cardiosphere Growth:

Following institutional guidelines, and with patient consent, human biopsy specimens were obtained from patients undergoing clinically-indicated percutaneous endomyocardial biopsy and processed as described (6) with modifications. Specimens consisted of whole or partial bioptome "bites", stored on ice in high-potassium cardioplegic solution and processed within two hours (Fig. 1A, step 1). Samples were cut into fragments from which gross connective tissue was removed. The fragments were then washed, partially-digested enzymatically, and the single cells discarded. The remaining tissue fragments were cultured as "explants" on dishes coated with fibronectin (Fig. 1A, step 2). After several days, a layer of stromal-like cells arose from adherent explants over which small, round, phase-bright cells migrated. Once confluent, the loosely-adherent cells surrounding the explants were harvested by gentle enzymatic digestion (Fig. 1A, step 3). These cells were seeded at 2-3x10⁴ cells/mL on poly-D-lysine-coated dishes in media designed for optimal growth of cardiospheres (Fig. 1A, step 4). Detached cardiospheres were then plated on fibronectin-coated flasks and expanded as adherent monolayers (Fig. 1A, step 5), which could be subsequently passaged by trypsinization. Single cells were counted under phase microscopy using a hemocytometer as cardiosphere-forming cells and during CDC passaging to track cell growth for each specimen. Isolation of the cardiosphere-forming cells was repeated up to 3 more times from the same specimen.

### Sub-population Selection and Flow Cytometric Analysis:

To characterize the antigenic features of cells that form cardiospheres, cells obtained during the first harvesting (Fig. 1A, 3) were sub-selected by magnetic-activated cell separation with an APC-conjugated monoclonal antibody against c-Kit, followed by labeling with a microbead-conjugated anti-APC, followed by separation using OctoMACS. CD105⁺ populations were then sub-selected with a second antibody directly conjugated to a microbead.

CDCs were passaged two times as adherent monolayers and then used for flow cytometry experiments. c-Kit-APC, CD105-PE, and similarly conjugated isotype-matched control monoclonal antibodies were utilized. Gates were established by 7-AAD fluorescence and forward scatter. Data were collected using a FACScalibur cytofluorometer with CellQuest software.

### Adenovirus Creation and Cell Transduction:

The E. coli β-galactosidase (lacZ) gene was cloned into an adenoviral shuttle vector pAd-Lox to generate pAd-Lox-LacZ by Cre-Lox recombination in Cre-4 293HEK cells as described (9). CDCs were passaged two times and transduced with virus as adherent monolayers. Transduction efficiencies of 90% were achieved with an MOI of 20 for 12 hours.

### Myocardial Infarction and Cell Injection:

Adenovirally-transduced CDCs were injected into adult male SCID-beige mice 10-16 weeks of age. Myocardial infarction (MI) was created by ligation of the mid-left anterior descending coronary artery as described (10) and cells or vehicle injected under direct visualization at two peri-infarct sites. CDCs (10⁵) were injected in a volume of 10 µL of PBS (5 µL at each site), with 10⁵ primary human skin fibroblasts or 10 µL of PBS as controls. All mice underwent echocardiography prior to surgery (baseline) and again 20 days post-surgery. Ejection fractions (EFs) were calculated using V1.3.8 software from 2D long-axis views taken through the infarcted area. Mice were then euthanized at 0, 8, or 20 days, and the excised hearts prepared for histology.

### Immunostaining, Immunohistochemistry, and Microscopy:

Cardiospheres were collected for immunostaining when they had reached 100-1000 cells in size. Primary antibodies against c-Kit, CD105, cardiac myosin heavy chain (cMHC), and cardiac troponin I (cTnI) were used for immunostaining. Secondary antibodies conjugated with Alexa fluorochromes were utilized. Immunostaining was performed as previously described (6). Confocal fluorescence imaging was performed on an Eclipse TE2000-U equipped with a krypton/argon laser using UltraVIEW software.

Mouse hearts were excised, embedded in OCT compound, frozen, and sectioned in 5 µm slices. Tissue sections were stained with hematoxylin-eosin and b-galactosidase reagent or Masson's trichrome (11). Tissue viability within the infarct zone was calculated from Masson's trichrome stained sections (12, 13) by tracing the infarct borders manually and then using ImageJ software to calculate the percent of viable myocardium within the overall infarcted area, as demonstrated in Figure S1.

### Statistics:

All results are presented as means ±SEM. The significance of differences between any two groups was determined by the Student's t-test. Multiple groups were compared using GB-Stat software using one-way ANOVA and group pairs compared by the Bonferroni-Dunn method if a significant F value was obtained. A value of p<0.05 was considered significant.

The generalized estimation equation (GEE) approach was employed (14) to identify parameters that were independently associated with high cell yield. Data from patients who donated multiple specimens were treated as repeated measures. Those parameters that were significant (p≤0.1) in the univariate models were included in the final, multivariate models. The analysis was performed with the use of SAS software. A final value of p<0.05 was considered significant. All p-values reported are 2-sided.

**Table 1. Products and Manufacturers.**

| | | | |
|---|---|---|---|
| *Media Recipes:* | | | |
| Explant and CDC media: IMDM, 20% FBS, 1% penicillin-streptomycin, 1% L-glutamine, 0.1mM 2-mercaptoethanol | | | |
| Cardiosphere media: 35% IMDM and 65% DMEM/F-12 Mix, 3.5% FBS, 1% penicillin-streptomycin, 1% L-glutamine, 0.1mM 2-mercaptoethanol, thrombin, B-27, bFGF, EGF, and Cardiotrophin-1 at final working concentrations | | | |
| | | | |

| *Product:* | *Working concentration:* | *Manufacturer:* | |
|---|---|---|---|
| IMDM | | Invitrogen | |
| DMEM/F-12 Mix | | Invitrogen | |
| Thrombin | 1 unit/mL | Sigma | |
| B-27 | 1:50 | Invitrogen | |
| bFGF | 80 ng/mL | PeproTech | |
| EGF | 25 ng/mL | PeproTech | |
| Cardiotrophin-1 | 4 ng/mL | PeproTech | |
| Fibronectin | 25 µg/mL | BD Biosciences | |
| Poly-D-lysine | 20 µg/mL | BD Biosciences | |
| c-Kit-APC | 1:10 | BD Pharmingen | |
| CD105 MicroBeads | 1:5 | Miltenyi Biotec | |
| Anti-APC MultiSort | 1:4 | Miltenyi Biotec | |
| CD105-PE | 1:10 | R&D Systems | |
| 7-AAD | 20 µg/mL | Calbiochem | |
| SCID-beige mice | | Harlan | |
| Dermal fibroblasts | | ATCC | |
| c-Kit pAb | 1:100 | Abcam | |
| CD105 mAb | 1:50 | R&D Systems | |
| cMHC pAb | 1:100 | (6) Rome, Italy | |
| cTnI mAb | 1:200 | Chemicon | |
| Alexa 488, 568 | 1:400 | Invitrogen | |
| OCT | | VWR Scientific | |
| | | | |
| *Equipment and Software:* | | *Manufacturer:* | |
| OctoMACS | | Miltenyi Biotec | |
| FACScalibur | | BD Biosciences | |
| Vevo 660 Echo | | VisualSonics | |
| Eclipse TE2000-U | | Nikon | |
| CellQuest | | BD Biosciences | |
| V1.3.8 software | | VisualSonics | |
| UltraVIEW software | | Perkin Elmer | |
| ImageJ software | | NIH | |
| GB-Stat v10 | | Dynamic Microsystems Inc. | |
| SAS software v9.1 | | SAS Institute Inc. | |

### EXAMPLE 2

### Specimen Processing and Cardiosphere-forming Sub-populations:

Figure 1B shows a typical explant, after mincing and partial enzymatic digestion, on the day it was obtained and also on days 3 (Fig. 1C) and 13 (Fig. 1D), immediately prior to first harvest. Harvesting of cardiosphere-forming cells (Fig. 1A, step 3) was initially performed 8 or more days after obtaining a specimen and at 4-12 day intervals thereafter. Panel E summarizes the results of sub-population selection experiments performed using cells harvested from 3 different patient specimens. The large majority of the cells that generate cardiospheres are CD105⁺, those that are c-Kit⁺ and those that are c-Kit⁻. Typical cardiospheres are shown in Fig. 1F, 12 days after harvest. Floating cardiospheres were plated for expansion (Fig. 1A, step 5) 4-28 days after step 3 and passaged at 2-7 day intervals thereafter. Figure 1G shows CDCs plated on fibronectin during expansion at passage 2, when those cells were harvested for injection.

### EXAMPLE 3

### Patient Specimens and Cardiosphere Growth:

83 patient specimens (21.0±1.9 mg) were obtained for analysis. 72 of the specimens were obtained from patients who had received a heart transplant and 11 were from patients awaiting transplant. Nine transplanted patients donated multiple specimens. 78 of 83 specimens were processed, and 4 of those specimens never harvested were from repeat patients, yielding growth data from 69 of 70 patients. Cumulative growth curves for each specimen are depicted in Figure 1, Panels H and I. The growth curves from patients awaiting transplant (Fig. 1H) are similar to those from transplanted patients (Fig. 1I), showing a wide range of growth potential among specimens. Patient parameters are summarized in Table 2 for the non-transplanted and transplanted groups. A GEE analysis involving all patient parameters listed in the table revealed no independent predictors for high cell yield within the non-transplanted group. Within the transplanted group, specimens from patients with a higher EF tended to yield more cells, but the effect was weak (R²=??, final estimate=0.04, p<0.05).

**Table 2. Patient Population Summary.**

| | *Non-transplanted Patients:* | *Transplanted Patients:* |
|---|---|---|
| Patient age | 47.2±3.7 years | 53.6±1.7 years |
| Patient sex | 63% male, 37% female | 73% male, 27% female |
| Patient ejection fraction | 36.9±4.7% | 61.9±0.8% |
| Donor age | | 31.4±1.6 years |
| Donor sex | | 69% male, 31 % female |
| Time out from transplant | | 4.5±0.6 years |
| Donor ischemic time | | 173.9±7.8 minutes |
| Pathological rejection level* | | 0.5±0.1 |
| Immunosuppressive level** | | 31% normal, 43% low, 26% high |
| * grade 0=0, grade 1A=0.5, grade 1B=1, grade 2=2, grade 3A=3 | | |
| ** considered for Cyclosporine and FK506 (± Rapamycin) relative to time out from transplant (24, 25) | | |

### EXAMPLE 4

### Cardiosphere and Cardiosphere-derived Cell Phenotypes:

The rationale for using CDCs lies in the unique biology of cardiospheres and their cell progeny. The self-organizing cardiospheres create a niche environment favoring the expression of stem cell antigens (e.g., c-Kit and CD105, Fig. 3A) and frequently manifest a surface phenotype marked by mature cardiac-specific antigens (cMHC and cTnI, Fig. 3B) with retention of internal "stemness". In fact, c-Kit and CD105 were present in all cardiospheres examined (10 or more from each of 10 patients), with c-Kit either localized to the core or expressed throughout the sphere, and CD105 typically localized to the periphery or expressed throughout. CDCs after two passages retain high levels of c-Kit and CD105 antigen expression (Fig. 3C, representative of expression profiles of CDCs from 3 and 2 different patients respectively).

### EXAMPLE 5

### Cardiosphere-derived Cell Engraftment, Regeneration, and Functional Improvement:

CDCs from 4 different patients were utilized for in vivo experiments. To assess engraftment and cell migration, mice were injected with lac-Z-expressing CDCs and sacrificed at each of 3 time points (0, 8, and 20 days following injection). At day 0, CDCs were located at injection sites in the border zone, but at day 8 and day 20 injected cells were distributed mainly within the MI area, forming islands or continuous bands of β-galactosidase positive tissue (Fig. 5).

Eight mice were injected with CDCs and followed for 20 days; 11 mice served as controls (4 with fibroblasts, and 7 with PBS). Figure 4A shows a typical β-galactosidase staining pattern indicating the distribution of injected human cells after 20 days in vivo. Note the band of blue cells infiltrating the infarct zone, which was not apparent in the fibroblast-injected mice (Fig. 4B) or the PBS-injected mice. Masson's trichrome-stained sections were used to quantify regeneration (Fig. 4, C and D) as illustrated in the Supplement. Panel C, from a CDC-injected heart, shows a number of obvious red regions within the blue infarct zone; fewer such regions are evident in the fibroblast-injected heart (Fig. 4D). CDC-injected mice had a higher fraction of viable fuchsin-positive tissue within the MI zone (24.9±1.1%) compared to fibroblast-injected mice (17.7±1.8%, p<0.01) or PBS-injected mice (13.7±0.7%, p<0.01), but the overall total infarct area was similar to that in the two control groups (60.6±6.4 CDC, 76.9±7.0 fibroblast, 75.7±2.7 PBS, units in 10⁴ pixels; p=NS). The differences between the CDC group and each of the control groups in percent viable myocardium within the MI zone, 7.2% and 11.2%, represent the extents of myocardial regeneration attributable to the CDCs.

Echocardiograms were performed for all groups at 20 days; Figure 5 shows examples from the CDC and fibroblast-treated groups at end-diastole and end-systole. Pooled data for left ventricular EF (LVEF, Fig. 5E) and left ventricular fractional area (LVFA, Fig. 5F) reveal a higher LVEF in the CDC-treated group (38.8±1.7%) as compared to either the fibroblast-treated (24.5±1.8%, p<0.01) or the PBS-treated group (26.4±3.0%, p<0,01), but the two control groups were indistinguishable. There was no difference among the LVEFs at baseline.

### EXAMPLE 6

### Process for the Isolation of Cardiac Stem Cells from Cardiac Biopsy Specimens

Pluripotent stem cells may be isolated from cardiac biopsy specimens or other cardiac tissue using a multi-step process (see fig 1a for schematic). First, cardiac tissue is obtained via percutaneous endomyocardial biopsy or via sterile dissection of the heart. Once obtained, tissue specimens are stored on ice in a high-potassium cardioplegic solution (containing 5% dextrose, 68.6mmol/L mannitol, 12,5meq potassium chloride, and 12.5meq sodium bicarbonate, with the addition of 10 units/mL of heparin) until they are processed (up to 12 hours later). For processing, specimens are cut into 1-2 mm³ pieces using sterile forceps and scissors; any gross connective tissue is removed. The fragments are then washed with Ca⁺⁺-Mg⁺⁺-free phosphate buffered saline (PBS) and typically digested for 5 min at room temperature with 0.05% trypsin-EDTA. Alternatively the tissue fragments may be digested in type IV collagenase (1 mg/mL) for 30 minutes at 37°C. Preliminary experiments have shown that cellular yield is greater per mg of explant tissue when collagenase is used.

Once digestion is complete, the remaining tissue fragments are washed with "Complete Explant Medium" (CEM) containing 20% heat-inactivated fetal calf serum, 100 Units/mL penicillin G, 100 µg/mL streptomycin, 2 mmol/L L-glutamine, and 0.1mmol/L 2-mercaptoethanol in Iscove's modified Dulbecco medium to quench the digestion process. The tissue fragments are minced again with sterile forceps and scissors and then transferred to fibronectin-coated (25 µg/mL for ≥ 1 hour) tissue culture plates, where they are placed, evenly spaced, across the surface of the plate. A minimal amount of CEM is added to the plate, after which it is incubated at 37°C and 5% CO₂ for 30 minutes to allow the tissue fragments, now referred to as "explants", to attach to the plate (fig 1b). Once the explants have attached, enough CEM is added to the plate to cover the explants, and the plates are returned to the incubator.

After a period of 8 or more days, a layer of stromal-like cells begins to arise from adherent explants, covering the surface of the plate surrounding the explant. Over this layer a population of small, round, phase-bright cells is seen (fig 1c,d). Once the stromal cell layer becomes confluent and there is a large population of bright phase cells, the loosely-adherent cells surrounding the explants are harvested. This is performed by first washing the plate with Ca⁺⁺-Mg⁺⁺-free PBS, then with 0.48 mmol/L EDTA (for 1-2 min) and finally with 0.05% trypsin-EDTA (for 2-3 min). All washes are performed at room temperature under visual control to determine when the loosely adherent cells have become detached. After each step the wash fluid is collected and pooled with that from the other steps. After the final wash, the explants are covered again with CEM and returned to the incubator. Each plate of explants may be harvested in this manner for up to four times at 5-10 day intervals. The pooled wash fluid is then centrifuged at 1000 rpm for 6-8 minutes, forming a cellular pellet. When centrifugation is complete, the supernatant is removed, the pellet is resuspended, and the cells are counted using a hemacytometer. The cells are then plated in poly-d-lysine coated 24-well tissue culture plates at a density ranging from 3-5 x 10⁴ cells/well (depending on the species) and returned to the incubator. The cells may be grown in either "Cardiosphere Growth Media" (CGM) consisting of 65% Dulbeco's Modified Eagle Media 1:1 with Ham's F-12 supplement and 35% CEM with 2% B27, 25 ng/mL epidermal growth factor, 80 ng/mL basic fibroblast growth factor, 4 ng/mL Cardiotrophin-1 and 1 Unit/mL thrombin, or in CEM alone.

In either media, after a period of 4-28 days, multicellular clusters ("cardiospheres") will form, detach from the tissue culture surface and begin to grow in suspension (fig 1e,f). When sufficient in size and number, these free-floating cardiospheres are then harvested by aspiration of their media, and the resulting suspension is transferred to fibronectin-coated tissue culture flasks in CEM (cells remaining adherent to the poly-D-lysine-coated dishes are not expanded further). In the presence of fibronectin, cardiospheres attach and form adherent monolayers of "Cardiosphere-Derived Cells" (CDCs) (fig 1g). These cells will grow to confluence and then may be repeatedly passaged and expanded as CDCs, or returned to poly-d-lysine coated plates, where they will again form cardiospheres. Grown as CDCs, millions of cells can be grown within 4-6 weeks of the time cardiac tissue is obtained, whether the origin of the tissue is human (fig 1i), porcine or from rodents (data not shown). When collagenase is used, the initial increase in cells harvested per mass of explant tissue results in faster production of large numbers of CDCs.

### References

The disclosure of each reference cited is expressly incorporated herein.

### References:

1. Quaini F, Urbanek K, Beltrami AP, et al. Chimerism of the transplanted heart. N Engl J Med 2002;346(1):5-15.
2. Beltrami AP, Barlucchi L, Torella D, et al. Adult cardiac stem cells are multipotent and support myocardial regeneration. Cell 2003;114(6):763-76.
3. Oh H, Bradfute SB, Gallardo TD, et al. Cardiac progenitor cells from adult myocardium: homing, differentiation, and fusion after infarction. Proc Natl Acad Sci U S A 2003;100(21):12313-8.
4. Urbanek K, Quaini F, Tasca G, et al. Intense myocyte formation from cardiac stem cells in human cardiac hypertrophy. Proc Natl Acad Sci U S A 2003;100(18):10440-5.
5. Urbanek K, Torella D, Sheikh F, et al. Myocardial regeneration by activation of multipotent cardiac stem cells in ischemic heart failure. Proc Natl Acad Sci U S A 2005;102(24):8692-7.
6. Messina E, De Angelis L, Frati G, et al. Isolation and expansion of adult cardiac stem cells from human and murine heart. Circ Res 2004;95(9):911-21.
7. Mason JW. Techniques for right and left ventricular endomyocardial biopsy. Am J Cardiol 1978;41(5):887-92.
8. Anastasiou-Nana MI, O'Connell JB, Nanas JN, Sorensen SG, Anderson JL. Relative efficiency and risk of endomyocardial biopsy: comparisons in heart transplant and nontransplant patients. Cathet Cardiovasc Diagn 1989;18(1):7-11.
9. Hoppe UC, Marban E, Johns DC. Distinct gene-specific mechanisms of arrhythmia revealed by cardiac gene transfer of two long QT disease genes, HERG and KCNE1. Proc Natl Acad Sci U S A 2001;98(9):5335-40.
10. Stull LB, Leppo MK, Szweda L, Gao WD, Marban E. Chronic treatment with allopurinol boosts survival and cardiac contractility in murine postischemic cardiomyopathy. Circ Res 2004;95(10):1005-11.
11. Biological Stain Commission., Conn HJ, Clark G. Staining procedures used by the Biological Stain Commission. 3d ed. Baltimore,: Published for the Biological Stain Commission by Williams & Wilkins; 1973.
12. Pfeffer MA, Pfeffer JM, Fishbein MC, et al. Myocardial infarct size and ventricular function in rats. Circ Res 1979;44(4):503-12.
13. Edelberg JM, Lee SH, Kaur M, et al. Platelet-derived growth factor-AB limits the extent of myocardial infarction in a rat model: feasibility of restoring impaired angiogenic capacity in the aging heart. Circulation 2002;105(5):608-13.
14. Zeger SL, Liang KY. Longitudinal data analysis for discrete and continuous outcomes. Biometrics 1986;42(1):121-30.
15. Schachinger V, Assmus B, Britten MB, et al. Transplantation of progenitor cells and regeneration enhancement in acute myocardial infarction: final one-year results of the TOPCARE-AMI Trial. J Am Coll Cardiol 2004;44(8):1690-9.
16. Strauer BE, Brehm M, Zeus T, et al. Repair of infarcted myocardium by autologous intracoronary mononuclear bone marrow cell transplantation in humans. Circulation 2002;106(15):1913-8.
17. Fernandez-Aviles F, San Roman JA, Garcia-Frade J, et al. Experimental and clinical regenerative capability of human bone marrow cells after myocardial infarction. Circ Res 2004;95(7):742-8.
18. Rubio D, Garcia-Castro J, Martin MC, et al. Spontaneous human adult stem cell transformation. Cancer Res 2005;65(8):3035-9.
19. Menasche P, Hagege AA, Vilquin JT, et al. Autologous skeletal myoblast transplantation for severe postinfarction left ventricular dysfunction. J Am Coll Cardiol 2003;41(7):1078-83.
20. Siminiak T, Kalawski R, Fiszer D, et al. Autologous skeletal myoblast transplantation for the treatment of postinfarction myocardial injury: phase I clinical study with 12 months of follow-up. Am Heart J 2004;148(3):531-7.
21. Smits PC, van Geuns RJ, Poldermans D, et al. Catheter-based intramyocardial injection of autologous skeletal myoblasts as a primary treatment of ischemic heart failure: clinical experience with six-month follow-up. J Am Coll Cardiol 2003;42(12):2063-9.
22. Urbanek K, Rota M, Cascapera S, et al. Cardiac Stem Cells Possess Growth Factor-Receptor Systems That After Activation Regenerate the Infarcted Myocardium, Improving Ventricular Function and Long-Term Survival. Circ Res 2005.
23. Limana F, Germani A, Zacheo A, et al. Exogenous High-Mobility Group Box 1 Protein Induces Myocardial Regeneration After Infarction via Enhanced Cardiac C-Kit+ Cell Proliferation and Differentiation. Circ Res 2005.
24. Taylor DO, Barr ML, Radovancevic B, et al. A randomized, multicenter comparison of tacrolimus and cyclosporine immunosuppressive regimens in cardiac transplantation: decreased hyperlipidemia and hypertension with tacrolimus. J Heart Lung Transplant 1999;18(4):336-45.
25. Kobashigawa J, Miller L, Renlund D, et al. A randomized active-controlled trial of mycophenolate mofetil in heart transplant recipients. Mycophenolate Mofetil Investigators. Transplantation 1998;66(4):507-15.

### CLAUSES

1. A method of increasing function of a damaged or diseased heart of a mammal, comprising:
   administering to the mammal a population of cells, whereby the population of cells increases cardiac function in the mammal, wherein the population of cells is obtained by the process of culturing cells obtained from cardiospheres on a surface as a monolayer.
2. The method of clause 1 wherein the cells are administered systemically.
3. The method of clause 2 wherein the cells migrate to the damaged or diseased heart and engraft to the diseased heart.
4. The method of clause 2 wherein the cells migrate to the damaged or diseased heart and provide diffusible products to the diseased heart.
5. The method of clause 1 wherein the cells are administered locally.
6. The method of clause 1 wherein the mammal has chronic cardiac disease.
7. The method of clause 1 wherein the mammal has experienced an acute cardiac disease.
8. The method of clause 1 wherein the cells are cultured in the absence of exogenous growth factors EGF and bFGF, cardiotrophin-1, and thrombin.
9. The method of clause 1 wherein the cardiospheres are formed in the absence of exogenous growth factors EGF and bFGF, cardiotrophin-1, and thrombin.
10. The method of clause 1 wherein the cells are cultured in the presence of fetal bovine serum but in the absence of other exogenous growth factors.
11. The method of clause 1 wherein the cardiospheres are formed in the presence of fetal bovine serum but in the absence of other exogenous growth factors.
12. The method of clause 1 wherein the cells are allogeneic to the mammal.
13. The method of clause 1 wherein the cells are autologous to the mammal.
14. A method of increasing function of a damaged or diseased heart of a mammal comprising:
   administering to the mammal a population of *in vitro-*expanded cells, wherein the cells have the capacity to form cardiospheres in suspension culture, but wherein the cells are not in the form of cardiospheres when administered.
15. The method of clause 14 wherein the cells are administered systemically.
16. The method of clause 14 wherein the cells are administered locally.
17. The method of clause 14 wherein the mammal has chronic cardiac disease.
18. The method of clause 14 wherein the damaged or diseased heart has experienced an acute cardiac disease.
19. The method of clause 14 wherein the cells are allogeneic to the mammal.
20. The method of clause 14 wherein the cells are autologous to the mammal.
21. A method of treating a mammal with a damaged or diseased heart, comprising: obtaining heart tissue from the damaged or diseased heart of the mammal or from a healthy heart of a donor via a percutaneous endomyocardial biopsy; treating the heart tissue to obtain and expand a population of cardiac stem cells; introducing the cardiac stem cells and/or their progeny into the damaged or diseased heart of the mammal.
22. The method of clause 21 wherein the first and second mammals are two individuals and the cells are allogeneic to the second mammal.
23. The method of clause 21 wherein the first and second mammals are one individual and the cells are autologous to the mammal.
24. The method of clause 21 wherein the heart tissue is obtained from the crista terminalis within the heart.
25. The method of clause 21 wherein the heart tissue is obtained from the right ventricular endocardium within the heart.
26. The method of clause 21 wherein the heart tissue is obtained from the septal or ventricle wall.
27. The method of clause 21 wherein the heart tissue is obtained from the atrial appendages.
28. The method of clause 21 wherein the step of treating employs incubation of the heart tissue with a protease and collection of cardiac stem cells thereby released from the heart tissue to form the population of cardiac stem cells.
29. The method of clause 28 wherein the protease is collagenase.
30. The method of clause 21 wherein the step of treating employs culturing in suspension to obtain cardiospheres.
31. The method of clause 21 wherein the step of treating employs culturing in suspension to obtain cardiospheres followed by monolayer growth on a surface.
32. The method of clause 21 wherein the step of obtaining employs a bioptome comprising a flexible catheter.
33. The method of clause 21 wherein the step of reintroducing employs systemic administration of the cardiac stem cells.
34. The method of clause 21 wherein the step of reintroducing employs localized administration to the heart.
35. The method of clause 21 wherein the step of reintroducing is performed by needle injection into the heart wall by catheter or direct visualization.
36. The method of clause 21 wherein the step of reintroducing is via coromary arteries.
37. A method of treating a cardiac biopsy specimen, comprising:
   incubating the cardiac biopsy specimen in the presence of a protease;
   collecting the cells liberated from the biopsy specimen by the protease incubation;
   culturing the cells on a surface as a monolayer to expand number of cells.
38. The method of clause 37 further comprising the step of growing the cells in suspension culture whereby they form cardiospheres.
39. The method of clause 37 further comprising the step of introducing the cells into a mammal.
40. The method of clause 39 wherein the cells are allogeneic to the mammal.
41. The method of clause 39 wherein the cells are autologous to the mammal.
42. The method of clause 37 wherein the protease is collagenase.
43. A method of treating a mammal with a damaged or diseased organ, comprising:
   obtaining tissue from the damaged or diseased organ of the mammal or from a healthy organ of a donor via a percutaneous biopsy;
   treating the tissue to obtain and expand a population of stem cells;
   introducing the stem cells and/or their progeny into the damaged or diseased organ of the mammal.
44. The method of clause 43 wherein the organ is a kidney.
45. The method of clause 43 wherein the cells are allogeneic to the mammal.
46. The method of clause 43 wherein the cells are autologous to the mammal.
47. A method for expanding a population of cardiac stem cells, comprising:
   disaggregating one or more first cardiospheres to individual cells or smaller aggregates of cells;
   culturing said individual cells or smaller aggregates of cells on a surface as a monolayer.
48. The method of clause 47 wherein the disaggregating is performed by trituration.
49. The method of clause 47 wherein the culturing is performed in the presence of fetal bovine serum but in the absence of other exogenous growth factors.
50. The method of clause 47 further comprising the step of incubating at least some of the cells of the monolayer in suspension culture whereby they form one or more second cardiospheres.
51. The method of clause 50 further comprising the step of disaggregating one or more second cardiospheres to individual cells or smaller aggregates of cells.
52. A population of *in vitro*-expanded cells in a monolayer, wherein the cells have the capacity to form cardiospheres in suspension culture, but which are not in the form of cardiospheres.
53. A population of cells made by the process of culturing cells on a surface as a monolayer, wherein the cells are obtained from disaggregated cardiospheres.
54. The method of clause 1, 14, or 21 wherein the cells are transfected with an expression construct comprising a coding sequence for a protein selected from the group consisting of:
   akt, connexin 43, other connexins, HIF1α, VEGF, FGF, PDGF, IGF, SCF, myocardin, cardiotrophin, L-type calcium channel α subunit, L-type calcium channel β subunit, and Nkx2.5.
55. The population of clause 52 or 53 wherein the cells are transfected with an expression construct comprising a coding sequence for a protein selected from the group consisting of: akt, connexin 43, other connexins, HIF1α, VEGF, FGF, PDGF, IGF, SCF, myocardin, cardiotrophin, L-type calcium channel α subunit, L-type calcium channel β subunit, and Nkx2.5.
56. The method of clause 1, 31, 37, 47, or 53 wherein the surface is coated with fibronectin.
57. The method of clause 1, 14, 21, 39, or 43 wherein the mammal is a human.
58. The method of clause 1, 14, 21, 39, or 43 wherein the mammal is a dog.
59. The method of clause 1, 14, 21, 39, or 43 wherein the mammal is a horse.
60. The method of clause 1, 14, 21, 37, 43, or 47 wherein the cells are human cells.
61. The method of clause 1, 14, 21, 37, 43, or 47 wherein the cells are horse cells.
62. The method of clause 1, 14, 21, 37, 43, or 47 wherein the cells are dog cells.
63. The method of clause 1, 14, 21, 37, 43, or 47 wherein the cells are pig cells.
64. The population of clause 52 or 53 wherein the cells are human cells.
65. The population of clause 52 or 53 wherein the cells are horse cells.
66. The population of clause 52 or 53 wherein the cells are dog cells.
67. The population of clause 52 or 53 wherein the cells are pig cells.
68. A method of treating a kidney biopsy specimen, comprising:
   incubating the kidney biopsy specimen in the presence of a protease;
   collecting the cells liberated from the biopsy specimen by the protease incubation;
   culturing the cells on a surface as a monolayer to expand number of cells.
69. The method of clause 68 further comprising the step of introducing the cells into a mammal.
70. The method of clause 69 wherein the cells are allogeneic to the mammal.
71. The method of clause 69 wherein the cells are autologous to the mammal.
72. The method of clause 68 wherein the protease is collagenase.

## Claims

1. An allogeneic population of cardiac stem cells derived from human biopsies without antigenic selection of stem cell markers, wherein said allogeneic population is suitable for use in the repair of damaged cardiac tissue, comprising:
cardiosphere derived cells (CDCs) isolated from a cardiac tissue of a first subject and expanded in culture without antigenic selection of stem cell markers;
wherein said CDCs express stem cell markers and exhibit regenerative potential such that said CDCs facilitate formation of myocardium and improve cardiac function;
wherein said CDCs are sized for systemic delivery and migration to the heart;
wherein said CDCs are suitable for administration to a second subject that is allogeneic with respect to said first subject.

2. The CDCs of Claim 1, wherein prior to expansion in culture, the cardiac tissue is partially digested to generate an explant, and single cells resulting from the partial digestion are discarded.

3. The CDCs of any one of the preceding Claims, wherein said CDCs generate cytokines, chemokines, or other diffusible factors that yield improvements in the growth or function of said damaged cardiac tissue.

4. The CDCs of any one of the preceding claims, wherein said CDCs are less than about 100 microns in size.

5. The CDCs of any one of the preceding claims, wherein the systemic administration route is selected from the group consisting of intravenous injection, intraarterial injection, perfusion, and infusion.

6. The CDCs of Claim 1, wherein said CDCs are also suitable for local administration.

7. The CDCs of any one of the preceding claims, wherein administration of said CDCs to said second subject results in engraftment of said CDCs in the cardiac tissue of said second subject, and wherein said engraftment persists for at least about 20 days.

8. The CDCs of any one of the preceding claims, wherein administration of said CDCs results in a functional improvement in said damaged cardiac tissue.

9. The CDCs of Claim 9, wherein said functional improvement comprises an increase in left ventricular function.

10. The CDCs of any one of the preceding claims, wherein administration of said CDCs results in improved cardiac cell viability.

11. The CDCs of any one of the preceding claims, wherein said damaged cardiac tissue results from myocardial infarction and wherein administration of said CDCs results in a decrease in infarct size.

12. The CDCs of any one of the preceding claims, wherein said cardiac tissue of said first subject is obtained from the crista terminalis, right ventricular endocardium, septal wall, ventricle wall, and/or atrial appendages.

13. The CDCs of any one of the preceding claims, wherein said CDCs are suitable for administration to said second subject in an amount between, between 1 x 10⁶ and 10 x 10⁶ cells per kg of body weight of the second subject.

14. Use of the CDCs of any one of the preceding claims in the manufacture of a medicament for treating a damaged or diseased heart of a mammal.

15. A method of generating the CDCs of any one of Claims 1 to 14, comprising:
digesting cardiac tissue obtained from a mammal to generate digested cardiac tissue;
culturing said digested tissue on a surface to generate a layer of stromal-like cells over which loosely adherent spherical phase-bright cells migrate;
harvesting said loosely adherent spherical phase-bright cells;
culturing said harvested spherical phase-bright cells on a surface to generate cardiospheres;
isolating said cardiospheres;
culturing said cardiospheres on a surface comprising fibronectin;
allowing said cultured cardiospheres to form adherent monolayers on said surface, thereby generating said CDCs.
